# FASCICULE DE BREVET EUROPEEN

(11) **EP 0 631 776 B1**
(45) Date de publication et mention de la délivrance du brevet: **22.11.2000**
(21) Numéro de dépôt: 94401460.4
(22) Date de dépôt: 28.06.1994
(51) Int. Cl.: A61K 31/175, A61K 31/165, A61K 31/12

(54) **Utilisation de dérivés de la bêta-naphtoquinone pour l'accélération de la prolifération des cellules endothéliales et de l'inhibition des NO synthases**
Verwendung von Beta-Naphthochinonderivaten zur Beschleunigung der Proliferation der Endothelzellen und Inhibierung der NO-Synthasen
Use of beta-naphthoquinone for accelerating the proliferation of endothelial cells and inhibition of NO synthases

(30) Priorité: 02.07.1993 FR 9308111
(43) Date de publication de la demande: 04.01.1995
(73) Titulaire: HOECHST MARION ROUSSEL, 92800 Puteaux (FR)
(72) Inventeur: Bloy, Christian, F-75015 Paris (FR); Cazenave, Jean-Pierre, F-67085 Strasbourg Cédex (FR); Hercelin, Bernard, F-60600 Clermont (FR); Teisseire, Bernard, F-75019 Paris (FR)
(74) Mandataire: Vieillefosse, Jean-Claude

(56) Documents cités:
- FR-M- 924
- LA GAZETTE MEDICALE, vol.100, no.20, 27 Mai 1993 page 38 PLOIN, M. 'ETIOVEN 10mg, M DICAMENT DE L'ANN E'
- AGRESSOLOGIE, vol.12, no.1, 1971 pages 25 - 30 LABORIT, H. ET AL 'EFFETS DE LA MONO-SEMICARBAZONE DE LA BETA-NAPHTOQUINONE DANS LE CHOC H MORRAGIQUE EXP RIMENTAL'

## Description

La présente invention concerne une nouvelle utilisation de dérivés de la béta-naphtoquinone ainsi que de leurs sels.

Le brevet Spécial de Médicament 924 M décrit l'application à titre de médicaments de dérivés de la béta-naphtoquinone. Selon ce brevet, ces dérivés sont présentés comme possédant des propriétés hémostatiques et des propriétés vitaminiques.

On sait que l'endothélium est constitué par une mono-couche de cellules d'origine mésodermique qui tapisse l'intérieur de tout le système vasculaire. Il constitue donc l'interface entre le sang et les tissus avoisinants. Cette localisation lui confère un rôle majeur dans le maintien de l'intégrité vasculaire et sanguine.

Le détachement de la monocouche de cellules endothéliales non thrombogènes entraîne l'exposition du sous-endothélium thrombogène (adhésion plaquettaire et leucocytaire). L'exposition du sous-endothélium est une situation pathologique qui se retrouve dans certaines interventions chirurgicales : embolectomies, angioplasties, endartériectomie et greffes de prothèses vasculaires ou dans certaines situations de pathologie médicale (athérosclérose, vascularites). Toutes ces situations ont un intérêt commun qui est la reconstitution rapide d'une surface endothéliale intacte et fonctionnelle. Il a été démontré récemment que les facteurs de croissance polypeptidiques (FGF : fibroblast growth factor ; PDGF : platelet derived growth factor ou EGF : epidermal growth factor) jouent un rôle important dans la réparation de lésions vasculaires ou cutanées : ulcères, escarres et brûlures.

En poursuivant ses études sur les dérivés de la béta-naphtoquinone précités, la demanderesse vient de trouver de façon tout à fait inattendue que ces derniers présentaient une remarquable activité permettant d'accélérer la prolifération des cellules endothéliales et d'inhiber les NO synthases constitutive et induite.

La présente demande a ainsi pour objet une nouvelle utilisation de dérivés de la béta-naphtoquinone de formule générale (I) : 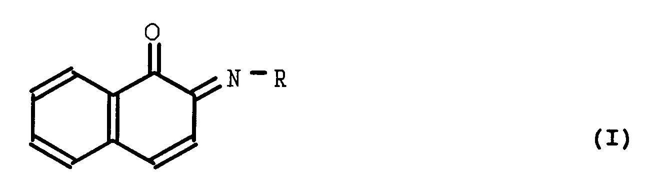 dans laquelle R représente un groupement de formule -NH-CO-NH₂, ou un groupement de formule -NH-CO-CH₃, ou un groupement hydroxyle, ainsi que leurs sels d'addition avec les acides minéraux ou organiques pharmaceutiquement acceptables, pour la fabrication d'un médicament permettant d'accélérer la prolifération des cellules endothéliales et d'inhiber les NO synthases.

Les sels d'addition avec les acides minéraux ou organiques pharmaceutiquement acceptables peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques tels que les acides méthane et éthane sulfoniques et arylsulfoniques, tels que l'acide benzènesulfonique.

Parmi les dérivés de la béta-naphtoquinone de formule générale (I), on retient notamment :
- le produit de formule (I) dans laquelle R représente un groupement de formule -NH-CO-NH₂, ainsi que ses sels d'addition avec les acides minéraux ou organiques pharmaceutiquement acceptables,
- le produit de formule (I) dans laquelle R représente un groupement de formule -NH-CO-CH₃, ainsi que ses sels d'addition avec les acides minéraux ou organiques pharmaceutiquement acceptables,
- le produit de formule (I) dans laquelle R représente un groupement hydroxy, ainsi que ses sels d'addition avec les acides minéraux ou organiques pharmaceutiquement acceptables.

Parmi les produits préférés de l'invention, utilisés pour la fabrication d'un médicament permettant d'accélérer la prolifération des cellules endothéliales et d'inhiber les NO synthases, on retient tout particulièrement la 2-semicarbazone de la 1,2-naphtoquinone plus connue sous la dénomination commune internationale de naftazone. Dans la partie expérimentale, ce produit figure sous sa DCI (dénomination commune internationale).

En raison de ces remarquables propriétés permettant d'accélérer la prolifération des cellules endothéliales et d'inhiber les NO synthases, illustrées plus loin dans la partie expérimentale, les dérivés de la béta-naphtoquinone de formule (I), tels que définis ci-dessus, ainsi que leurs sels d'addition avec les acides minéraux ou organiques pharmaceutiquement acceptables, peuvent être utilisés, dans les greffes et les prothèses vasculaires, et dans le traitement de l'ensemble des processus de cicatrisation et réparation tissulaire.

La dose usuelle, variable selon le produit utilisé, le sujet traité et l'affection en cause,peut être par exemple de 1 mg à 100 mg par jour, par voie orale ou par voie injectable.

L'invention a également pour objet les compositions pharmaceutiques qui renferment à titre de principe actif l'un au moins des médicaments définis ci-dessus.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les divers agents mouillants, dispersants ou émulsifiants, les conservateurs.

Des procédés de préparation des dérivés de la béta-naphtoquinone de formule (I) ainsi que de leurs sels ont été décrits dans la littérature. On peut notamment citer les procédés décrits dans le brevet spécial de médicament 924M précité, ou dans le brevet français 2 103 504.

Il va être donné maintenant à titre non limitatif des exemples de compositions pharmaceutiques pouvant être utilisées dans la mise oeuvre de l'invention.

### EXEMPLE 1 :

On a préparé des comprimés répondant à la formule suivante :

| | |
|---|---|
| - Naftazone | 10 mg |
| - Excipient q.s.p. pour un comprimé terminé à | 150 mg |

(détail de l'excipient : lactose, amidon, talc, stéarate de magnésium).

### EXEMPLE 2 :

On a préparé un soluté injectable répondant à la formule suivante :

| | |
|---|---|
| - Naftazone | 5 mg |
| - Excipient aqueux stérile q.s.p. | 2 ml |

### EXEMPLE 3 :

On a introduit 8,6 g de Naftazone dans 200 ml d'anhydride acétique. Après 10 heures de chauffage à 140°C, puis refroidissement, filtration, extraction du précipité par le chloroforme, évaporation à sec, reprise par l'éthanol, passage sur charbon actif, filtration et cristallisation lente, on a obtenu 5,1 g de produit répondant à la formule (I) avec R = -NH-CO-CH₃ (poudre microcristalline jaune ocre, F = 137-138°C).

### ETUDE PHARMACOLOGIQUE

### I - Etude in vitro de la prolifération des cellules endothéliales humaines de veine saphène

Nous avons évalué les propriétés mitogéniques de la naftazone sur des cellules endothéliales humaines de veine saphène en culture dans des conditions standard.

La naftazone, à des concentrations comprises entre 10⁻⁵ M et 10⁻⁷ M, accélère la prolifération de cellules endothéliales, ensemencées à faible densité, d'un facteur 2 au maximum en comparaison du témoin (sans naftazone).

A confluence, la densité cellulaire reste légèrement augmentée (environ 20 %). Par contre, en présence de concentrations optimales de sérum, la différence de densité cellulaire entre le contrôle et les échantillons contenant la naftazone s'estompe.

En conclusion, dans les conditions de l'essai, l'effet de la naftazone reste en général inférieur ou égal à celui induit par les FGFS mais, à confluence, la densité cellulaire en présence des facteurs FGFs est nettement supérieure à celle obtenue avec la naftazone, en comparaison du témoin.

### II - Etude de l'inhibition de la NO synthase constitutive des cellules endothéliales vasculaires

La naftazone a fait l'objet d'études réalisées sur anneaux (3 à 4 mm de long) d'aorte abdominale de rat CD (200-250 g, Charles River, France) et de veine fémorale de lapin New-Zealand (2-2,5 kg, Charles River, France).

Les anneaux sont placés en chambre d'organe (25 ml, Phymep, France) dans une solution de Krebs-Ringer. Cette solution est tonométrée en permanence par un mélange gazeux à 95 % O₂ - 5 % CO₂, à pH 7,40 et thermostatée à 37°C. La force isométrique développée par les anneaux est mesurée par des capteurs de tension (Phymep, France). Les anneaux sont amenés à leur point optimal de la relation tension-longueur par des stimulations répétées à l'aide de KCl 40 mM. Les études pharmacologiques sont réalisées après rinçages et une période d'équilibre de 45 minutes. Lorsque la réponse pharmacologique du vaisseau est étudiée sans endothélium, celui-ci est éliminé par frottement de l'intérieur du vaisseau à l'aide d'une pince à dissection. L'absence d'endothélium est vérifiée par l'absence de relaxation du vaisseau induite par l'ACh 10⁻⁶ M après contraction à NAD 10⁻⁷ M. Les contractions sont exprimées en % de la contraction maximale à KCl 60 mM, les relaxations sont exprimées en % de la contraction à NAD 10⁻⁵ M ou PGF2^{α} 10⁻⁷ M.

Les propriétés pharmacologiques suivantes ont été observées :
1 - la naftazone (10⁻⁶ M - 10⁻⁴ M) augmente significativement le tonus basal des anneaux aortiques de rat et de veine fémorale de lapin.
2 - la naftazone (10⁻⁶ M - 10⁻⁴ M) potentialise les contractions obtenues avec la NAD (10⁻⁹ M - 10⁻⁵ M) et avec la 5HT (10⁻⁹ M - 10⁻⁵ M) de façon significative et dose dépendante sur aorte de rat. Cette potentialisation disparait en l'absence d'endothélium.
3 - Sur anneaux aortiques contractés à la NAD (10⁻⁵ M), la naftazone (10⁻⁶ M - 10⁻⁴ M) inhibe de façon significative et dose dépendante la relaxation à l'ACh (10⁻⁹ M - 10⁻⁵ M). L'inhibition atteint 60 à 80 % pour la naftazone (10⁻⁵ M) pour une relaxation obtenue avec ACh (10⁻⁵ M). Cette inhibition est comparable en cinétique, en amplitude, à celle obtenue avec le Nώ-Nitro-L-Arginine (10⁻⁶ M) (inhibiteur puissant des NO synthases).
   Sur des anneaux aortiques contractés à PGF2^{α} (2.10⁻⁶ M) en présence d'Indométhacine (10⁻⁵ M) et de Kétanserine (10⁻⁵ M), la naftazone (10⁻⁶ M - 10⁻⁴ M) inhibe la relaxation obtenue avec 5HT (10⁻⁹ M - 10⁻⁵ M) de façon significative et dose dépendante (10⁻⁶ M - 10⁻⁴ M).
4 - Sur anneaux aortiques contractés à la NAD (10⁻⁷ M) ou à PGF2^{α} (2.10⁻⁶ M) en présence d'Indométhacine (10⁻⁵ M), la naftazone inhibe la relaxation obtenue avec le calcium ionophore A23187 (10⁻⁹ M - 10⁻⁶ M), ceci de façon significative et dose dépendante (10⁻⁶ M - 10⁻⁴ M).
5 - L'inhibition de la relaxation endothélium dépendante à l'ACh, à la 5HT et au A23187 obtenue avec la naftazone (10⁻⁵ M) est levée totalement par la L-Arginine (9.10⁻⁴ M).
6 - La naftazone (10⁻⁵ M) inhibe de façon significative l'hyporéactivité à la NAD (10⁻⁹ M - 10⁻⁵ M) obtenue après 3 heures d'incubation d'anneaux aortiques de rat en présence de Lipopolysaccharides E. coli 011B4 (10 µm/ml). Cet effet est comparable à celui obtenu avec le Nώ-Nitro-L-Arginine (10⁻⁵ M).
7 - La naftazone (10⁻⁵ M) potentialise la contraction à la NAD (10⁻⁹ M - 10⁻⁵ M) obtenue sur anneaux de veine fémorale de lapin et inhibe la relaxation à l'ACh (10⁻⁹ M - 10⁻⁵ M).

En conclusion, l'augmentation du tonus basal, la poten tialisation des effets vasoactifs de la Noradrénaline, de la Sérotonine, l'inhibition des relaxations endothélium dépendantes à l'Acétylcholine, la Sérotonine et au Calcium ionophore A23187, la levée de l'inhibition des relaxations endothélium dépendantes par la L-Arginine, l'inhibition de l'hyporéactivité à la Noradrénaline induite par l'endotoxine bactérienne, font classer la naftazone comme inhibiteur de NO synthase constitutive et de NO synthase induite.

## Revendications

1. Utilisation de dérivés de la béta-naphtoquinone de formule générale (I) : 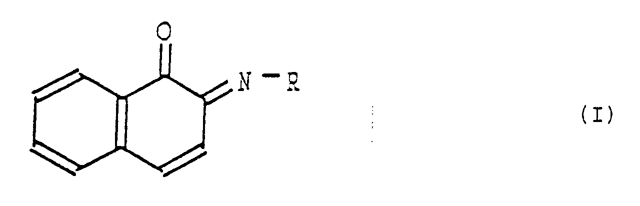 dans laquelle R représente un groupement de formule -NH-CO-NH₂, ou un groupement de formule -NH-CO-CH₃, ou un groupement hydroxyle, ainsi que leurs sels d'addition avec les acides minéraux ou organiques pharmaceutiquement acceptables, pour la fabrication d'un médicament permettant d'accélérer la prolifération des cellules endothéliales dans les greffes et les prothèses vasculaires, dans le traitement de l'ensemble des processus de cicatrisation et réparation tissulaires.

2. Utilisation selon la revendication 1, caractérisée en ce que le dérivé de la béta-naphtoquinone est un produit de formule générale (I) dans laquelle R représente un groupement de formule -NH-CO-NH₂, ainsi que ses sels d'addition avec les acides minéraux ou organiques pharmaceutiquement acceptables.

3. Utilisation selon la revendication 1, caractérisée en ce que le dérivé de la béta-naphtoquinone est un produit de formule générale (I) dans laquelle R représente un groupement de formule -NH-CO-CH₃, ainsi que ses sels d'addition avec les acides minéraux ou organiques pharmaceutiquement acceptables.

4. Utilisation selon la revendication 1, caractérisée en ce que le dérivé de la béta-naphtoquinone est un produit de formule générale (I) dans laquelle R représente un groupement hydroxy, ainsi que ses sels d'addition avec les acides minéraux ou organiques pharmaceutiquement acceptables.

5. Utilisation selon la revendication 1 ou 2, caractérisée en ce que le dérivé de la béta-naphtoquinone est la 2-semicarbazone de la 1,2-naphtoquinone.

## Claims

1. Use of derivatives of beta-naphthoquinone of general formula (I): 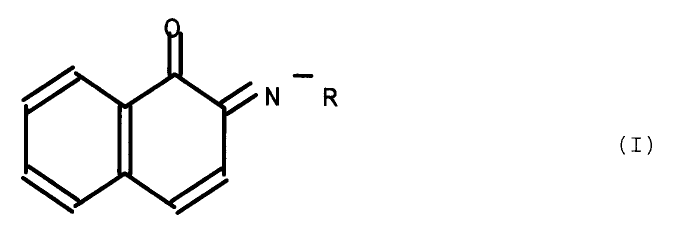 in which R represents a group of formula -NH-CO-NH₂, or a group of formula -NH-CO-CH₃, or a hydroxyl group, as well as their addition salts with pharmaceutically acceptable mineral or organic acids, for the production of a medicament allowing acceleration of the proliferation of endothelial cells in vascular transplants and prostheses, in the treatment of the whole cicatrization and tissue repair process.

2. Use according to claim 1, characterized in that the derivative of beta-naphthoquinone is a product of general formula (I) in which R represents a group of formula -NH-CO-NH₂, as well as its addition salts with pharmaceutically acceptable mineral or organic acids.

3. Use according to claim 1, characterized in that the derivative of beta-naphthoquinone is a product of general formula (I) in which R represents a group of formula -NH-CO-CH₃, as well as its addition salts with pharmaceutically acceptable mineral or organic acids.

4. Use according to claim 1, characterized in that the derivative of beta-naphthoquinone is a product of general formula (I) in which R represents a hydroxy group, as well as its addition salts with pharmaceutically acceptable mineral or organic acids.

5. Use according to claim 1 or 2, characterized in that the derivative of beta-naphthoquinone is 1,2-naphthoquinone 2-semicarbazone.

## Patentansprüche

1. Verwendung von beta-Naphthochinon-Derivaten der allgemeinen Formel (I): 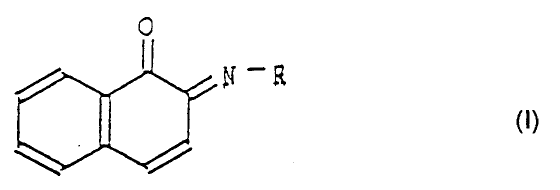 in der R eine Gruppe der Formel -NH-CO-NH₂ oder eine Gruppe der Formel -NH-CO-CH₃ oder eine Hydroxygruppe darstellt, sowie deren Additionssalzen mit den pharmazeutisch annehmbaren mineralischen oder organischen Säuren zur Herstellung eines Medikaments, das es ermöglicht, die Proliferation der Endothelzellen in den vaskulären Transplantaten und Prothesen zu beschleunigen, bei der Behandlung der Gruppe der Gewebevernarbungs- und -heilungsprozesse.

2. Verwendung gemäß Anspruch 1, dadurch gekennzeichnet, dass das beta-Naphthochinon-Derivat ein Produkt der allgemeinen Formel (I) ist, in der R eine Gruppe der Formel -NH-CO-NH₂ darstellt, sowie seiner Additionssalze mit den pharmazeutisch annehmbaren mineralischen oder organischen Säuren.

3. Verwendung gemäß Anspruch 1, dadurch gekennzeichnet, dass das beta-Naphthochinon-Derivat ein Produkt der allgemeinen Formel (I) ist, in der R eine Gruppe der Formel -NH-CO-CH₃ darstellt, sowie seiner Additionssalze mit den pharmazeutisch annehmbaren mineralischen oder organischen Säuren.

4. Verwendung gemäß Anspruch 1, dadurch gekennzeichnet, dass das beta-Naphthochinon-Derivat ein Produkt der allgemeinen Formel (I) ist, in der R eine Hydroxygruppe darstellt, sowie seiner Additionssalze mit den pharmazeutisch annehmbaren mineralischen oder organischen Säuren.

5. Verwendung gemäß Anspruch 1 oder 2, dadurch gekennzeichnet, dass das beta-Naphthochinon-Derivat 1,2-Naphthochinon-2-semicarbazon ist.
